# EUROPEAN PATENT SPECIFICATION

(11) **EP 3 651 818 B1**
(45) Date of publication and mention of the grant of the patent: **11.01.2023**
(21) Application number: 18752275.0
(22) Date of filing: 11.07.2018
(51) Int. Cl.: A61L 31/08

(54) **METHOD FOR TREATING AN ARTICLE MADE OF FIBROUS MATERIAL, ARTICLE OBTAINED BY SAID METHOD AND MEDICAL AND/OR HEALTH CARE AND/OR PERSONAL CARE DEVICE COMPRISING SAID ARTICLE**
VERFAHREN ZUR BEHANDLUNG EINES ARTIKELS AUS FASERMATERIAL, DURCH DIESES VERFAHREN HERGESTELLTER ARTIKEL UND MEDIZINISCHE UND/ODER GESUNDHEITSPFLEGE- UND/ODER KÖRPERPFLEGEVORRICHTUNG MIT DIESEM ARTIKEL
PROCÉDÉ DE TRAITEMENT D'UN ARTICLE EN MATÉRIAU FIBREUX, ARTICLE OBTENU PAR LEDIT PROCÉDÉ ET DISPOSITIF MÉDICAL ET/OU DE SOINS DE SANTÉ ET/OU D'HYGIÈNE PERSONNELLE COMPRENANT LEDIT ARTICLE

(30) Priority: 13.07.2017 IT 201700078999
(43) Date of publication of application: 20.05.2020
(73) Proprietor: Materias S.r.l., 80122 Napoli (NA) (IT)
(72) Inventor: NICOLAIS, Luigi, 80056 Ercolano (NA) (IT); D'ANNA, Andrea, 80146 Napoli (IT); DE FALCO, Gianluigi, 83100 Avellino (IT); COMMODO, Mario, 80146 Napoli (IT); MINUTOLO, Patrizia, 80146 Napoli (IT); SQUILLACE, Antonino, 80146 Napoli (IT); DEL GAUDIO, Pasquale, 84012 Agri (SA) (IT)
(74) Representative: PGA S.p.A.
(86) International application number: PCT/IB2018/055113
(87) International publication number: WO 2019/012448

(56) References cited:
- WO-A2-2010/077665
- DE-A1-102012 003 943
- US-A- 5 698 177
- US-A1- 2006 141 015
- ANTONIO TRICOLI ET AL: "Flame spray pyrolysis synthesis and aerosol deposition of nanoparticle films", AI CH E JOURNAL, vol. 58, no. 11, 17 February 2012 (2012-02-17), pages 3578-3588, XP055473329, US ISSN: 0001-1541, DOI: 10.1002/aic.13739
- MANJITH KUMAR ET AL: "Flame synthesis and characterization of nanocrystalline titania powders", PROCESSING AND APPLICATION OF CERAMICS, vol. 6, no. 3, 1 January 2012 (2012-01-01) , pages 165-171, XP055473407, ISSN: 1820-6131, DOI: 10.2298/PAC1203165K

## Description

The present invention relates to a method for treating an article made of fibrous material, able to provide said article with antibacterial and/or antifungal properties by means of the application on the surface of said article of particles of a titanium-based compound, preferably titanium dioxide (TiO2). Disclosed but not part of the invention as claimed, is an article obtained by means of said method and a medical and/or health care and/or personal care device comprising said article.

In environments such as hospitals it is extremely important to contrast the proliferation of fungi and bacteria, inasmuch as they are pathogenic factors especially for in-patients, out-patients and health care professionals. Among the devices that are most at risk of facilitating such proliferation are those made of fabric (for example sheets or white coats, but also disposable devices such as gauze or bandages), wherein fungi and bacteria can become ensconced and reproduce very rapidly. Over the years, consequently, progressively more attention has been paid to the development of technical solutions that would make it possible to reduce the vulnerability of fabric devices to the lodging and proliferation of fungi and bacteria.

Among these technical solutions, some of those deemed to be of greatest interest use titanium dioxide, in view of the undoubted cleansing and sanitising properties of this material. Known technical solutions wherein titanium dioxide particles are used entail their local application. To the surfaces of the fabrics to be treated, a solution of titanium dioxide in a solvent is applied. The solvent is then made to evaporate and particles of titanium dioxide thus remain positioned on the treated surfaces.

While such local application of particles of titanium dioxide improves the hygiene of the fabrics, it has undoubted critical issues. In the first place, application of the solvent and the subsequent applying of heat can damage the fabrics. In the second place, the process that allows the local application of titanium dioxide is poorly repeatable and is distinguished by a rather low efficiency. In the third place, titanium dioxide particles, following the evaporation of the solvent, remain on fabrics in agglomerated form.

This latter critical issue certainly constitutes the most significant one. Agglomerates of titanium dioxide particles, being all of large size and differing from one another in structure and geometry, cannot arrange themselves on the surface of the fabrics, to constitute a continuous, uniform coating layer. Between the agglomerates, wide interstices are created, i.e. wide areas that in fact are not treated and thus are highly susceptible being attacked by fungi and bacteria and hence to facilitate their proliferation. Moreover, because of their significant mass, agglomerates of titanium dioxide particles are easily removed by a mechanical action. Therefore, a simple friction of the fabrics after the local application of titanium dioxide particles causes their detachment and consequently the loss of all the fabric cleansing and sanitisation benefits. Lastly, because of the interstices between agglomerates, the chemical agents that come in contact with the fabrics (for example in a subsequent washing treatment) have facilitated paths to infiltrate between the titanium dioxide particles and hence to cause their detachment by chemical action.

The document DE102012003943A1 describes a method for applying a nano-layer with antimicrobial capabilities on a substrate of fabric, paper or generally of a fibrous materials, under atmospheric pressure conditions (thus avoiding the complexity and costs of vacuum processes). In the nano-layer is present a metal or a metal oxide (preferably ZnO, but also TiO2) in the form of nanoparticles generated by flame synthesis starting from a precursor compound. A nano-layer of metal or metal oxide nanoparticles can be applied to a garment for patients with lesions, to make it dermo-compatible and protective.

A purpose of the present invention is to solve the aforesaid drawbacks noted in relation to the prior art, in particular in relation to the local application of titanium dioxide particles.

Another purpose of the present invention is to make available a method for treating an article made of fibrous material (for example an article made of fabric or paper) distinguished by high hygiene.

Another purpose of the present invention is to make available a method for treating an article made of fibrous material that makes said article particularly capable of contrasting the proliferation of fungi and bacteria, both immediately after the execution of the treatment, and after the treatment as time goes by.

Another purpose of the present invention is to make available a method for treating an article made of fibrous material wherein the effectiveness of the treatment is not then compromised by mechanical actions (for example, friction) which the article undergoes after the treatment.

Another purpose of the present invention is to make available a method for treating an article made of fibrous material wherein the effectiveness of the treatment is not as easily compromised by the chemical agents with which the article comes in contact after the treatment (the article is then able to maintain appreciable resistance to fungi and bacteria even after undergoing some washing treatments).

Another purpose of the present invention is to attenuate the biological hazard to which are subject, in a hospital environment, both patients and health care professionals.

Another purpose not part of the present invention is to obtain sterile articles for the treatment, for example, of wounds with exudate, configured to minimise the risk of fungal or bacterial infections through the wounds.

These and other purposes are achieved by means of the method for treating an article made of fibrous material, by means of the fibrous article and by means of the medical and/or health care and/or personal care device according to one or more of the following aspects.

A first aspect of the invention refers to a method for treating an article made of fibrous material, adapted to provide said article with antibacterial and/or antifungal properties by means of the application on a surface of said article of particles of a metal oxide, preferably of a titanium- or zinc- or iron- or silver-based oxide, still more preferably titanium dioxide (TiO2), said method comprising the steps of:
i) generating said particles through Flame Synthesis of said particles from a precursor compound, preferably titanium tetraisopropoxide (TTIP) and/or titanium tetrachloride (TiCl4), and
ii) depositing in a controlled manner said particles on a surface of said article (9) by thermophoresis.

A second aspect of the invention, dependent on the first aspect, refers to a method for treating an article made of fibrous material, wherein said flame synthesis occurs by means of a reactor to which said precursor compound is carried and wherein said surface of said article is exposed to the output section of said reactor.

A third aspect of the invention, dependent on the second aspect, refers to a method for treating an article made of fibrous material, wherein the exposure of said surface of said article to said output section of said reactor is a periodic exposure wherein a first exposure phase is alternated with a second non-exposure phase, the duration of said first exposure phase being appreciably shorter than the duration of said second non-exposure phase.

A fourth aspect of the invention, dependent on the third aspect, refers to a method for treating an article made of fibrous material, wherein said duration of said first exposure phase is shorter than 1 second, preferably shorter than 0.1 seconds, yet more preferably approximately 0.02 seconds.

A fifth aspect of the invention, dependent on the third aspect or on the fourth aspect, refers to a method for treating an article made of fibrous material, wherein the duration of said periodic exposure is between 10 seconds and 10 minutes, preferably between 1 minute and 5 minutes, still more preferably between 150 seconds and 180 seconds.

A sixth aspect of the invention, dependent on any aspect between the third aspect and the fifth aspect, refers to a method for treating an article made of fibrous material, wherein said periodic exposure is obtained by rotation of a carousel, said article being applied to said carousel so as to assure the exposure of said surface of said article to said output section of said reactor.

A seventh aspect of the invention, dependent on any aspect between the second aspect and the sixth aspect, refers to a method for treating an article made of fibrous material, wherein said precursor compound is carried to said reactor by means of a solution of said precursor compound in a solvent, preferably ethanol.

An eighth aspect of the invention, dependent on any aspect between the second aspect and the seventh aspect, refers to a method for treating an article made of fibrous material, wherein said precursor compound is carried to said reactor in aerosol form, said aerosol being preferably generated by means of a vibrating orifice aerosol generator (VOAG).

A ninth aspect of the invention, dependent on the eighth aspect, refers to a method for treating an article made of fibrous material, wherein said aerosol is mixed in said reactor with a fuel, preferably ethylene premixed with air.

A tenth aspect of the invention, dependent on any aspect between the second aspect and the ninth aspect, refers to a method for treating an article made of fibrous material, wherein said precursor compound is carried to said reactor by saturation of a dispersion medium, preferably air, of said precursor compound and/or of said solution of said precursor compound in said solvent.

An eleventh aspect of the invention, dependent on any aspect between the second aspect and the tenth aspect, refers to a method for treating an article made of fibrous material, wherein the temperature of the flame at said output section of said reactor is between 1000 K and 2500 K, preferably between 1500 K and 2000 K, still more preferably between 1700 K and 1800 K and/or wherein the temperature difference between said article and the flame at said output section of said reactor is between 400 K and 2200 K, preferably between 400 K and 1700 K, still more preferably between 400 K and 1500 K.

A twelfth aspect of the invention, dependent on any of the preceding aspects, refers to a method for treating an article made of fibrous material, wherein said step i) is preceded by the preliminary step of regulating the numerousness of said particles and/or the coalescence of said particles and/or the possible agglomeration of said particles and/or the crystalline group of said particles.

A thirteenth aspect of the invention, dependent on the twelfth aspect, refers to a method for treating an article made of fibrous material, wherein in said preliminary step an adjustment of the distance of said surface of said article from said output section of said reactor is carried out, in particular of the component of said distance orthogonal to said output section of said reactor, an increase of said distance determining an increase of the size of said particles and/or the passage of said particles from the condition of non-agglomeration to the condition of agglomeration.

A fourteenth aspect of the invention, dependent on the twelfth aspect or on the thirteenth aspect, refers to a method for treating an article made of fibrous material, wherein in said preliminary step an adjustment of the concentration of said precursor compound in said solution is carried out, an increase of said concentration determining an increase of the numerousness of said particles and/or of the size of said particles and/or the passage of said particles from the condition of non-agglomeration to the condition of agglomeration.

A fifteenth aspect of the invention, dependent on any aspect between the twelfth aspect and the fourteenth aspect, refers to a method for treating an article made of fibrous material, wherein in said preliminary step an adjustment of the stoichiometry of the flame is carried out, in particular by adjustment of the quantity of comburent and/or of fuel sent into said reactor, a change of the stoichiometry of the flame determining a change of the quantity of oxygen present in the environment of the reactor and hence of the crystalline group of said particles.

A sixteenth aspect of the invention, dependent on any of the preceding aspects, refers to a method for treating an article made of fibrous material, wherein said particles are deposited on said surface of said article in the non-agglomeration condition.

A seventeenth aspect of the invention, dependent on any of the preceding aspects, refers to a method for treating an article made of fibrous material, wherein said particles are deposited on said surface of said article in a size of between 1 nm and 50 nm, preferably between 3 nm and 10 nm, still more preferably between 4 nm and 5 nm.

An eighteenth aspect of the invention, dependent on any of the preceding aspects, refers to a method for treating an article made of fibrous material, wherein said particles are deposited on said surface of said article crystallised in the tetragonal crystalline system, preferably in the form of anatase.

A nineteenth aspect of the invention, dependent on any of the preceding aspects, refers to a method for treating an article made of fibrous material, further comprising the step of:
iii) irradiating said surface of said article with an ultraviolet radiation (UV).

A twentieth aspect of the invention, dependent on the nineteenth aspect, refers to a method for treating an article made of fibrous material, wherein the duration of said step iii) is between 1 minute and 20 minutes, preferably between 3 minutes and 10 minutes, still more preferably approximately 5 minutes.

A twenty-first aspect but not part of the invention refers to an article made of fibrous material treated by means of the method according to any of the preceding aspects.

A twenty-second aspect but not part of the invention, dependent on the twenty-first aspect, refers to an article made of fibrous material, wherein said fibrous material comprises a synthetic material, preferably polypropylene and/or viscose, or cotton or paper.

A twenty-third aspect but not part of the invention, dependent on the twenty-first aspect or on the twenty-second aspect, refers to an article of fibrous material, wherein said fibrous material comprises a nonwoven fabric (NWF).

A twenty-fourth aspect but not part of the invention, dependent on the twenty-first aspect, refers to an article of fibrous material, wherein said fibrous material comprises a ceramic material or a metallic material.

A twenty-fifth aspect but not part of the invention refers to a medical and/or health care and/or personal care device, for example a sheet, a white coat, a mask, a bonnet, a bandage, a gauze, comprising an article according to any of the aspects of the twenty-first aspect to the twenty-fourth aspect.

The aspects of the present invention will become more readily apparent from the detailed description that follows, which will refer to the figures accompanying the following description, in which:
- Figure 1 schematically shows a system configured to implement the method according to the present invention;
- Figure 2 is an axonometric view of a component of the system in Figure 1;
- Figure 3 schematically shows a detail of the system in Figure 1;
- Figures 4a - 4c show magnified views of the same detail of a textile article respectively before being treated thermally, after being treated thermally for a first time interval and after being treated thermally for a second time interval;
- Figures 5a - 5c show magnified view of the detail of a textile article of Figure 4a after being thermally treated for the aforesaid first time interval;
- Figures 6a - 6c show magnified view of the detail of a textile article of Figure 4a after being thermally treated for the aforesaid second time interval;
- Figures 7a and 7b show, in the form of histograms, the effectiveness of the method according to the present invention in relation to a particular fungus (Candida albicans);
- Figures 8a and 8b show, in the form of histograms, the effectiveness of the method according to the present invention in relation to a particular Gram-positive bacterium (Staphylococcus aureus);
- Figures 9a and 9b show, in the form of histograms, the effectiveness of the method according to the present invention in relation to a particular Gram-negative bacterium (Pseudomonas aeruginosa) and
- Figures 10a and 10b show, in the form of histograms, the effectiveness of the method according to the present invention in relation to a particular fungus (Candida albicans) by comparison with the effectiveness of the method according to the prior art in relation to the same fungus. The present invention and the scope thereof is defined by the appended claims. The more generic description of the invention is provided for illustrative purposes only. Embodiments not falling under these claims are for reference purposes only.

The idea at the basis of the present invention is to make an article made of fibrous material particularly high performing with regard to its resistance to fungi and/or bacteria, applying a coating layer on the outer surface thereof, exploiting the physical phenomenon of thermophoresis to deposit, on said surface, particles of a metal oxide generated by flame synthesis.

The method according to the invention is therefore suitable to provide an article of fibrous material with antibacterial and/or antifungal properties by means of two distinct steps and carried out in succession with a time separation of the order of milliseconds.

In the first step of the method, the metal oxide particles are generated by flame synthesis starting from a precursor compound. In an advantageous embodiment of the present invention, the metal oxide of which particles are generated by flame synthesis is a titanium or zinc or iron or silver oxide. For example, the metal oxide of which particles are generated by flame synthesis is titanium dioxide (TiO2): in this case, titanium tetraisopropoxide (TTIP) and/or titanium tetrachloride (TiCl4) can be used.

In the second step of the method, the metal oxide particles generated are deposited by thermophoresis on a surface of the article, thus providing the article with the desired antibacterial and/or antifungal properties.

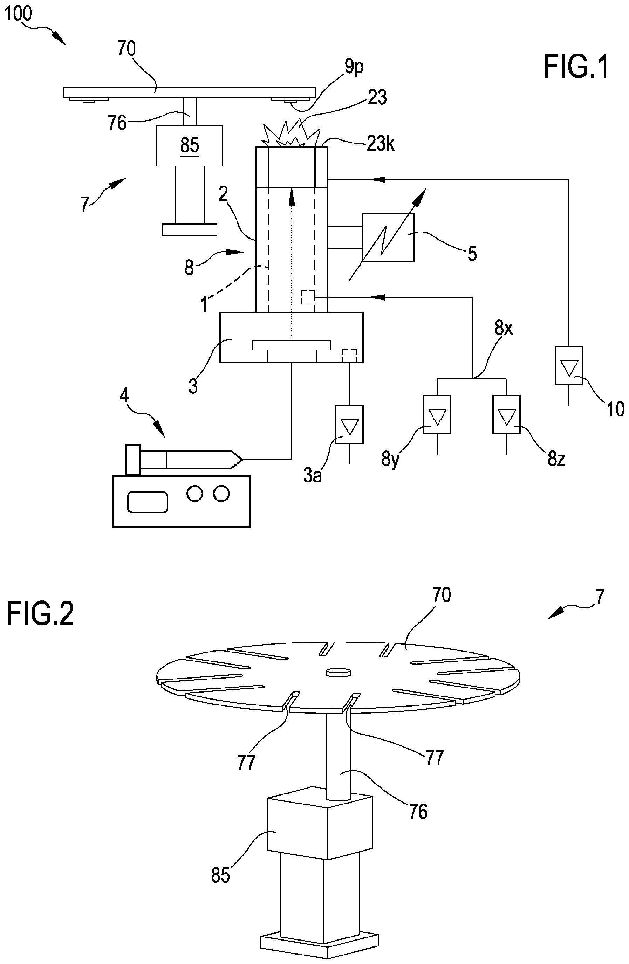

For the implementation of the present invention, the system 100 of Figure 1 can conveniently be used. Said system comprises a reactor 8 which, appropriately powered, makes possible to flame synthesise the particles of metal oxide for subsequent deposit on an article 9 of fibrous material. The reactor 8 is in particular a burner configured to generate a flame of sufficiently high temperature (of the order of 1800 k, or even higher) able to cause the synthesis of the metal oxide particles starting from the precursor compound, the latter having been carried to the reactor 8.

The article 9 is advantageously a medical and/or health care and/or personal care device, for example a device suitable to be used in hospital environments. In particular, the article 9 can comprise a sheet, a white coat, a mask, a bonnet, a bandage or a gauze. The article 9 can be a device usable a multiple number of times (having been adequately treated between one use and the next) or a disposable device. The fibrous material constituting the article 9 can comprise a synthetic material, preferably polypropylene and/or viscose, or cotton or paper. Alternatively, the fibrous material constituting the article 9 can comprise a nonwoven fabric (NWF). As an additional alternative, the fibrous material constituting the article 9 can comprise a ceramic material or a metallic material.

In the example illustrated in Figure 1, the reactor 8 is a honeycomb burner comprising two concentric tubes (inner tube 1 and outer tube 2) made of stainless steel that ends in an output section 8s from which the flame 23 is freed outside. To stabilise the flame 23 that is freed from the output section 8s and thus obtain a uniform radial effect, a cylinder of silicon carbide or of mullite (also with honeycomb configuration) is positioned in the upper part of the inner tube 1.

The flow of gas inside the reactor 8 can ideally be mono-dimensional (the flow develops mainly along the direction of development of the reactor 8) and laminar, the velocities of the gases being maintained intentionally low (approximately 0.1 m/s), to obtain at the output a stable, ideally unidirectional flow. The ideal unidirectionality of the flow of gas that traverses the output section 8s of the reactor 8 ensures that, arranging the article 9 so that the surface 9p thereof to be treated is exposed to the output section 8s, the particles of metal oxide that are generated by flame synthesis reach the surface 9p to be deposited thereon by thermophoresis, thus assuring a small dispersion of the particles and consequently a high efficiency of the treatment.

Observing the system of Figure 1, it is noticeable that the region upstream of the reactor 8 is occupied by a chamber 3 that is supplied with the precursor compound. The precursor compound is advantageously supplied through a solution of said precursor compound in a solvent. If titanium tetraisopropoxide (TTIP) and/or titanium tetrachloride (TiCl4) is used as a precursor compound, to obtain the synthesis of titanium dioxide (TiO2) particles, ethanol can conveniently be used as a solvent.

In an embodiment of the present invention, the precursor compound is supplied to the reactor 8 in aerosol form. Therefore the system 100 comprises an aerosol generator 4 which, in the example represented by way of non-limiting explanation in Figure 1, is of the vibrating orifice type. Said aerosol generator 4, developed on the basis of the studies by Berglund and Liu, is able to create monodispersed particles with size below 0.2 mm. An aerosol generator suitable for being used in the system 100 is marketed by TSI Incorporated ^{®} with the code 3450:
http://www.tsi.com/uploadedFiles/ Site Root/Products/Literature/Spec_Sheets/3450VibOr1930086RevA.pdf

The chamber 3 is supplied with the solution comprising the precursor compound and with a dispersing air flow coming from an appropriate supply 3a. The function of this air flow is to prevent or significantly to limit, inside the chamber 3, the coalescence of the particles of solution released in aerosol form in the chamber 3. The dispersing airflow, preferably saturated with particles of solution, then drags the particles of solution and contributes to convey them inside the reactor 8, where the particles of solution are then mixed to particles of a suitable fuel, for example a mixture of ethylene and air.

In an additional embodiment of the present invention (alternative or complementary with the preceding one), the precursor compound is supplied to the reactor by saturation of a dispersing medium, preferably air, of the precursor compound and/or of the solution of the precursor compound in the solvent.

Returning to the architecture of the system 100 represented in Figure 1, it is observed that the chamber 3 performs both the role of mixing chamber of the solution of the precursor compound in the solvent, because this solution tends to disperse uniformly in the dispersing air flow inside the chamber 3, and the role of solvent evaporation chamber, because the solvent in the chamber 3 starts to evaporate from the solution and therefore to free the precursor compound.

Immediately downstream of the chamber 3, in the system 100 the reactor 8 is supplied with the fuel and with the comburents necessary for generating the flame 23. In the example shown in Figure 1, it can be appreciated that, immediately downstream of the chamber 3, a supply 8x is provided, configured to supply a premixed mixture of fuel and comburent into the reactor 8. As a fuel, it is possible to use for example ethylene, coming from a first sub-supply 8y, and air, coming from a second sub-supply 8z. In this regard, it can be appreciated that the connection point between the first sub-supply 8y and the second sub-supply 8z is arranged at an adequate distance with respect to the point of supply of the mixture in the reactor 8. In this way, the fuel and the comburent are allowed to adequately mix together, so as to assure optimal flammability of the mixture. Moreover, regulating the quantities of fuel and/or comburent sent to the reactor 8 it is possible to modify the stoichiometry of the flame 23, obtaining conditions of excess fuel with respect to the stoichiometric, conditions of excess comburent with respect to the stoichiometric, or stoichiometric conditions according to the need.

By means of the architecture of the system 100 of Figure 1, a mixture is created, within the reactor 8, of fuel (ethylene, but also the ethanol freed from the solution with the precursor compound) and comburent (air and/or oxygen) that is quite homogeneous and that transports within it homogeneously dispersed particles of precursor compound. To raise the temperature of the aforesaid mixture along the reactor 8, between the inner tube 1 and the outer tube 2 is advantageously installed an electrical resistor 5 configured to heat the inner tube 1 and hence by convection the flow within the reactor 8.

The temperature distribution in the reactor 8 is such that the maximum temperature is recorded at the flame 23, where temperatures of the order of 1700 K - 1800 K can be reached, i.e. the optimal temperature for completion of the synthesis of the metal oxide, in particular titanium dioxide (TiO2) starting from the precursor compound. More generally, the temperature of the flame 23 at the output section 8s of the reactor 8 is between 1000 K and 2500 K, preferably between 1500 K and 2000 K, still more preferably between 1700 K and 1800 K. Comparing the temperature of the flame 23 at the output section 8s of the reactor 8, it is observed that the difference between said temperature is between 400 K and 2200 K, preferably between 400 K and 1700 K, still more preferably between 400 K and 1500 K. Since the precursor compound is carried in the flow ideally in the form of monodispersed particles, it is appreciated that the metal oxide particles that are created by flame synthesis are also monodispersed and have size of the order of 1 nm (de facto having zero mass).

To obtain an appropriate insulation of the flame 23 from the surrounding air, preventing the flame 23 from being perturbed or the mixture from being polluted by external contaminants, a flow of inert gas is released starting from the annular section 23k that surrounds the output section 8s. As an inert gas, a noble gas, for example argon, coming from an appropriate supply 10, is advantageously used. A sort of gas blade is thus created, which performs an effective function of protecting the flame 23 from the exterior. The flame 23 thus obtained has a carpet conformation, a blue coloration and a height H of the order of 1 mm.

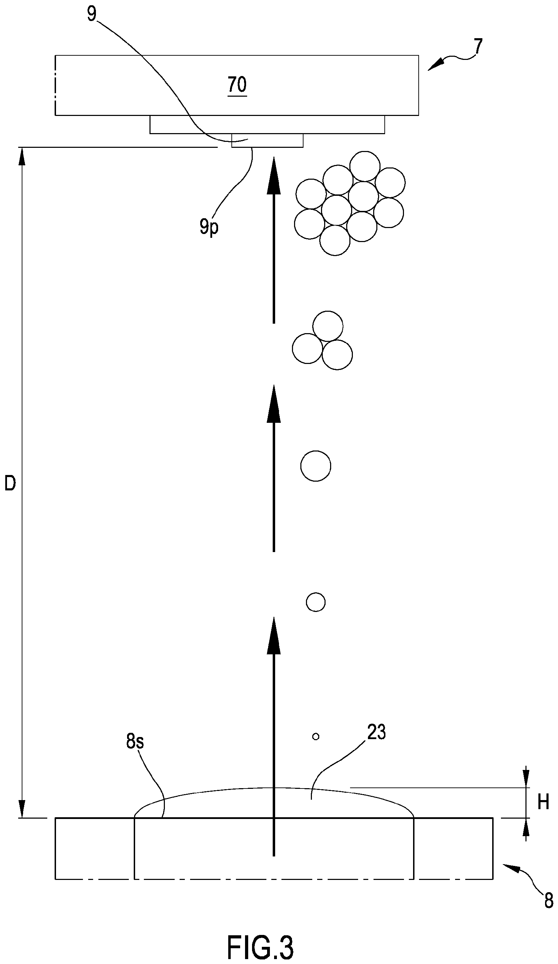

Once they are created by flame synthesis, the metal oxide particles move away from the reactor 8 until they are deposited by thermophoresis on the surface 9p of the article 9 made of fibrous material to be treated, said surface being exposed to the output section 8s of the reactor 8. Figure 3 shows what occurs to the metal oxide particles between their flame synthesis and their deposit on the surface 9p of the article 9.

In a first time interval starting from their creation (diameter 1 nm or smaller), the metal oxide particles tend at first to become larger by coalescence. Therefore, the size of the particles grows until reaching at first a value of 3 nm - 4 nm and then a value of 20 nm - 30 nm. Once a critical size is reached, in a second time interval the physical phenomenon of coalescence ceases to the advantage of the concurrent physical phenomenon of agglomeration. Metal oxide particles, then, instead of further increasing their size, tend to agglomerate forming clusters of metal oxide particles that are mutually diversified in terms of mass, dimensions and geometry.

Figure 3 therefore shows how, changing the distance D between the flame 23 and the surface 9p of the article 9 to be treated, the size of the metal oxide particles that will be deposited by thermophoresis on said surface 9p will consequently change as well. In addition, adjusting the distance D between the flame 23 and the surface 9p, so that it is smaller or larger than the critical distance starting from which the physical phenomenon of the agglomeration prevails over coalescence, the metal oxide particles will be deposited on the surface 9p of the article 9 mainly in the condition of non-agglomeration or mainly in the agglomeration condition.

Concerning the deposit of the metal oxide particles on the surface 9p, it is stressed that thermophoretic force does not depend on the mass of the particles, but only on the temperature difference between the temperature of the metal oxide particles (which are approximately at the temperature of the flame 23) and the temperature of the surface 9p of the article 9 (which is at ambient temperature or slightly above ambient temperature). Hence, although the metal oxide particles can differ from each other in terms of size and/or can be differently aggregated together, reach the surface 9p substantially with the same speed, therefore assuring an adequate uniformity of the coating layer of the surface 9p and consequently the desired increase of the antifungal and/or antibacterial properties of the article 9.

To provide the article 9 with the best antifungal and/or antibacterial properties, there is an interest in minimising the distance D between the flame 23 and the surface 9p of the article 9, so that metal oxide particles of the smallest possible size can be deposited on the surface 9p. In this way, the creation on the surface 9p of a coating layer that is as uniform, continuous and thin as possible is facilitated, so as to enhance the benefits of the method for treating the article 9 according to the present invention. In any case, it is preferable to prevent the distance D between the flame 23 and the surface 9p of the article 9 from exceeding the critical distance starting from which the agglomeration between the metal oxide particles prevails over coalescence. If agglomerates are deposited on the surface 9p, the substantial continuity of the coating layer would be compromised, so that areas susceptible to becoming lodging and proliferation areas for fungi and/or bacteria. Additionally, the possible deposit of agglomerates on the surface 9p would create evident non-uniformity in the application of the metal oxide to the article 9 and would also make it necessary to use a quantity of metal oxide particles that is markedly greater than the quantity necessary for the creation of a thin and uniform layer.

From what is described and illustrated in Figure 3, it follows that, to increase the effectiveness of the treatment method, it is absolutely advantageous to reduce the distance D between the flame 23 and the surface 9p of the article 9. On the other hand, however, said distance D must be sufficient to prevent the flame 23 from burning the article 9 made of fibrous material or to prevent the flame 23 from compromising the characteristics of the article 9.

To preserve the article 9 of fibrous material from possible damages due to its exposure to the flame 23, the method of the present invention can advantageously provide for the exposure of the surface 9p of the article 9 to the output section 8s of the reactor 8 to be achieved by means of a process of periodic exposure. Therefore a first phase in which the surface 9p of the article 9 is exposed to the output section 8s of the reactor 8 is then followed by a second phase in which the surface 9p of the article 9 is not exposed to the output section 8s of the reactor 8. The duration of the first exposure phase is appreciably shorter than the duration of the second non-exposure phase. In this way, it is possible to contain the heating of the surface 9p to less than 100 K (advantageously to approximately 50 K), so that the article 9 is not burned by the flame 23 and to avoid alterations of the characteristics of the fibrous material constituting the article 9. Advantageously, the duration of the first phase of exposure is shorter than 1 second, preferably shorter than 0.1 seconds, still more preferably approximately 0.02 seconds.

To allow an adequate quantity of metal oxide particles to be deposited on the surface 9p of the article 9, the aforesaid periodic exposure continues until a predetermined time threshold is reached. Advantageously, the periodic exposure of the surface 9p of the article 9 to the output section 8s of the reactor 8 has a duration t_{des} between 10 seconds and 10 minutes, preferably between 1 minute and 5 minutes, still more preferably between 150 seconds and 180 seconds.

In the example illustrated by way of non-limiting explanation in Figure 1, the periodic exposure of the surface 9p of the article 9 to the output section 8s of the reactor 8 is obtained by rotation of a carrousel 7, shown in detail in the axonometry of Figure 2. The carrousel 7 is configured so as to allow the application of the article 9 thereon and further to allow that the surface 9p of the article 9 thus applied to the carrousel 7 is adequately exposed to the output section 8s of the reactor 8. Advantageously, the carrousel 7 is configured to support the article 9 in a position located frontally to the output section 8s of the reactor 8, so that the metal oxide particles that are released by the reactor 8 (and that maintain, even after traversing the output section 8s, a direction of flow substantially parallel to the axis of the reactor 8) can more efficiently and easily reach the surface 9p.

The carrousel 7 comprises a table 70 that extends along a plane that is substantially parallel to the plane of lay of the output section 8s of the reactor 8. In the example represented in Figure 2, the table 70 has nearly circular shape. Although this shape is deemed to be the preferred shape, other shapes are nonetheless possible for the table 70 of the carrousel 7, for example a polygonal shape.

The table 70 is configured to rotate around its own axis. For this purpose, the table 70 is splined to a shaft 76 to which is coupled an electric motor 85. Table 70 then has at least a pair of slots 77 substantially parallel to each other, which extend along a substantially radial direction. The function of the slots 77 is to allow the article 9 to be stably supported by the table 70 of the carrousel 7. The article 9 is positioned adjacently to the surface of the carrousel 7 oriented towards the reactor 8 so that the two flaps of the article 9 are inserted into the slots 77. To the carrousel 7 are then applied fastening plates (not shown in Figure 2) that lock the flaps of the article 9, thus assuring that the article 9 is not applied loosely to the table 70 of the carrousel 7. Advantageously, the table 70 comprises a plurality of slots 77 (preferably equally distanced from each other) that make it possible to apply to the carrousel 7 a plurality of articles to be treated simultaneously.

According to the present invention, alternative devices to the carrousel 7 can be used to allow the periodic exposure of the surface 9p of the article 9 to the output section 8s of the reactor 8. Purely by way of explanatory but non-limiting example, one can mention, as alternatives to the carrousel 7, a wheel with orthogonal axis to the reactor 8 and supports for the articles obtained on the outer surface of the wheel or a roller mechanism configured to make the article 9 travel in closed loop trajectories.

An alternative embodiment of the present invention then a configuration is provided for the system 100 (in particular for the reactor 8) which makes it possible to generate the particles of a metal oxide by means of flame synthesis, changing the flow rates of fuel and of comburent sent to the reactor 8 and establishing (instead of laminar flow conditions) conditions of premixed flame or of flame with diffusion in turbulent flow.

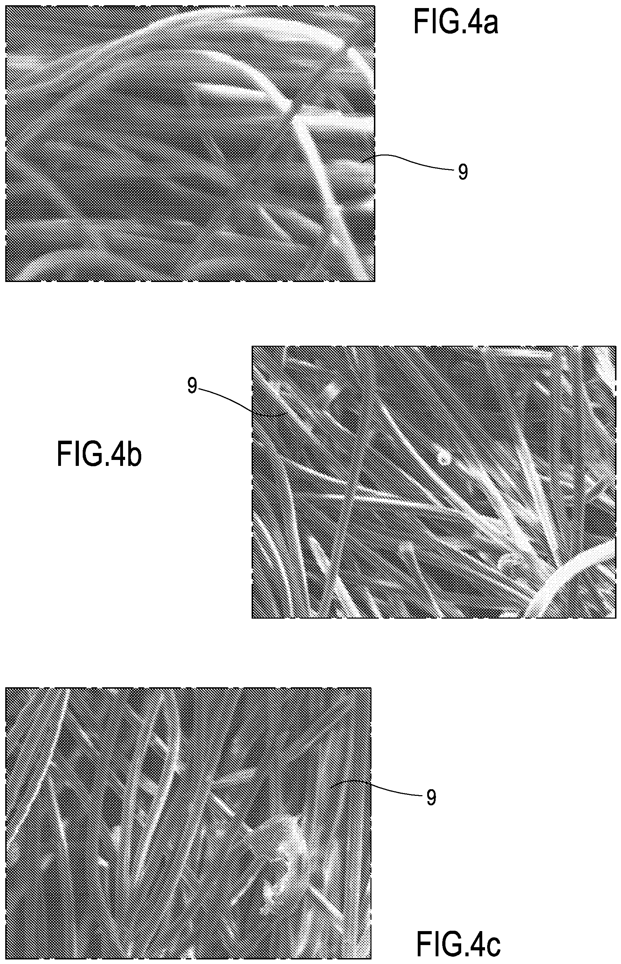

Figures 4a to 4c are photographic images of a detail of an article 9 made of fibrous material applied to the carrousel 7 and subjected to periodic exposure to the flame 23 described above, the fibrous material constituting the article 9 shown in the Figures being in particular a nonwoven fabric (NWF). Figure 4a shows said detail before subjecting the article 9 to periodic exposure to the flame 23. Figure 4b shows said detail after subjecting the article 9 to periodic exposure to the flame 23 for a duration t_{des} equal to 150 seconds. Figure 4c shows said detail after subjecting the article 9 to periodic exposure to the flame 23 for a duration t_{des} equal to 180 seconds. From a comparison between the three photographic images, it is readily apparent that the fibres that comprise the article 9 were not burned by periodic exposure to the flame 23 and that they have retained, without significant alteration, their own geometric and/or dimensional and/or mechanical characteristics.

Advantageously, the system 100 shown in Figure 1 comprises an electronic control unit, operatively connected to sensor devices and actuator devices. As sensor devices installable in the system 100 can be mentioned temperature sensors (adapted for example to measure the temperature of the flame 23) and/or chemical concentration sensors (adapted for example to measure the quantity of oxygen and/or of precursor compound inside the reactor 8). As actuator devices, instead, one can mention the actuators associated to the various supplies of the reactor 8 and/or the vibrating orifice of the aerosol generator 4. The function of the electronic control unit is to control the implementation of the method according to the invention by means of the system 100. An additional function of the electronic control unit is to assure that the design parameters match the desired parameters, to obtain the desired characteristics and/or values for the metal oxide particles that are deposited on the surface 9p of the article 9. There can be numerous adjustments in the system 100 allowed by the electronic control unit. For example, starting from the reading of the temperature of the flame 23, the electronic control unit can intervene on the electrical resistor 5 to increase and/or decrease the thermal power delivered by it and hence to increase and/or decrease the temperature of the flame 23.

In an embodiment of the present invention (not shown in the Figures accompanying the present description), the electronic control unit, to obtain for example the desired size of the metal oxide particles on the surface 9p of the article 9, can control actuating means interposed between the reactor 8 and the carrousel 7. The actuating means allow the reactor 8 and the carrousel 7 (and consequently the surface 9p of the article 9 and the output section 8s of the reactor 8) to approach or move away from each other, thus determining a decrease or an increase of the dimensions of the metal oxide particles deposited on the surface 9p. The actuating means can comprise, for example, a telescopic element applied to the shaft 76 to approach or remove the table 70 to and from the electric motor 85 and/or a carriage integral with the reactor 8 that allows it to slide along a fixed rail substantially parallel to the reactor 8, approaching it to or removing it from the table 70. The actuating means are operatively connected to the electronic control unit, so that the latter can finely control and/or adjust the distance between the table 70 and the output section 8s of the reactor 8 (and consequently the distance between the surface 9p of the article 9 and the output section 8s of the reactor 8). Advantageously, the electronic control unit allows the system 100 to implement a preliminary step to the method according to the invention, in which an adjustment is made to the numerousness of the metal oxide particles and/or the coalescence of the metal oxide particles and/or the possible agglomeration of the metal oxide particles and/or the crystalline group of the metal oxide particles. In particular:
i) the numerousness of the metal oxide particles that are deposited on the surface 9p of the article 9 can be adjusted by intervening on the duration t_{des} of the periodic exposure of the article 9 to the flame 23 (for example by prolonging said duration t_{des} from 150 seconds to 180 seconds, a higher number of metal oxide particles are deposited on the surface 9p, and consequently an improvement of the antifungal and/or antibacterial properties of the article 9, with the disadvantage of a longer treatment time and of higher consumption of precursor compound), but also intervening on the concentration of the precursor compound in the solution (starting from a reference concentration titanium tetraisopropoxide (TTIP) and/or of titanium tetrachloride (TiCl4) in ethanol, said reference concentration being for example equal to 0.3 M, the numerousness of the metal oxide particles that are deposited on the surface 9p can be increased adopting a higher concentration than the reference concentration, or reduced adopting a lower concentration than the reference concentration);
ii) the size of the metal oxide particles that are deposited on the surface 9p of the article 9 can be adjusted intervening on the distance between the surface 9p of the article 9 and the output section 8 of the reactor 8, or alternatively only on the component of said distance orthogonal to the output section 8s of the reactor 8 (increasing the distance between the surface 9p and the output section 8s causes a progressive increase of the size of the titanium oxide particles, since they are allowed more time to grow through the coalescence phenomenon), but also intervening on the concentration of the precursor compound in the solution (starting from a higher concentration of precursor compound in the solution, for equal distance between the surface 9p and the output section 8s metal oxide particles of larger size are obtained, because the greater numerousness of particles promotes the coalescence phenomenon);
iii) the condition of agglomeration or non-agglomeration of the metal oxide particles can be determined intervening on the distance between the surface 9p of the article 9 and the output section 8s of the reactor 8: therefore, to avoid the emergence (to a significant extent) of the agglomeration phenomenon, the distance between the surface 9p and the output section 8s is set to such a value that the metal oxide particles are not allowed to agglomerate, but only to grow by coalescence, the value of the distance between the surface 9p and the output section 8s to be set being the smaller, the greater the concentration of the precursor compound in the solution, given that an increase in the concentration of the precursor compound in the solution aids the agglomeration for the metal oxide particles;
iv) the crystalline group of the metal oxide particles is determined adjusting the stoichiometry of the flame 23, in particular adjusting the quantity of fuel and/or of comburent in the reactor 8, so that, changing the flow rate of ethylene and/or of air and/or of oxygen coming from the supply 3a and/or from the sub-supplies 8y and 8z, the concentration of oxygen present in the reaction environment can be changed, thus promoting the formation of different crystalline groups. In the example of use of titanium dioxide particles, it is possible to obtain the crystallisation of said compound in the three main crystalline group, i.e. rutile when the flame is operated in conditions of excess fuel, anatase when the flame is operated in conditions of excess comburent, and brookite when the flame is operated in stoichiometric conditions.

Advantageously, the method according to the present invention is implemented to deposit on the surface 9p of the article 9 the metal oxide particles in the condition of non-agglomeration. Still more advantageously, metal oxide particles are deposited on the surface 9p of the article 9 in the condition of non-agglomeration and with a dimension between 1 nm and 50 nm, preferably between 3 nm and 10 nm, still more preferably between 4 nm and 5 nm. Advantageously, metal oxide particles are deposited on the surface 9p of the article 9 crystallised in the tetragonal crystalline system, preferably in the form of anatase.

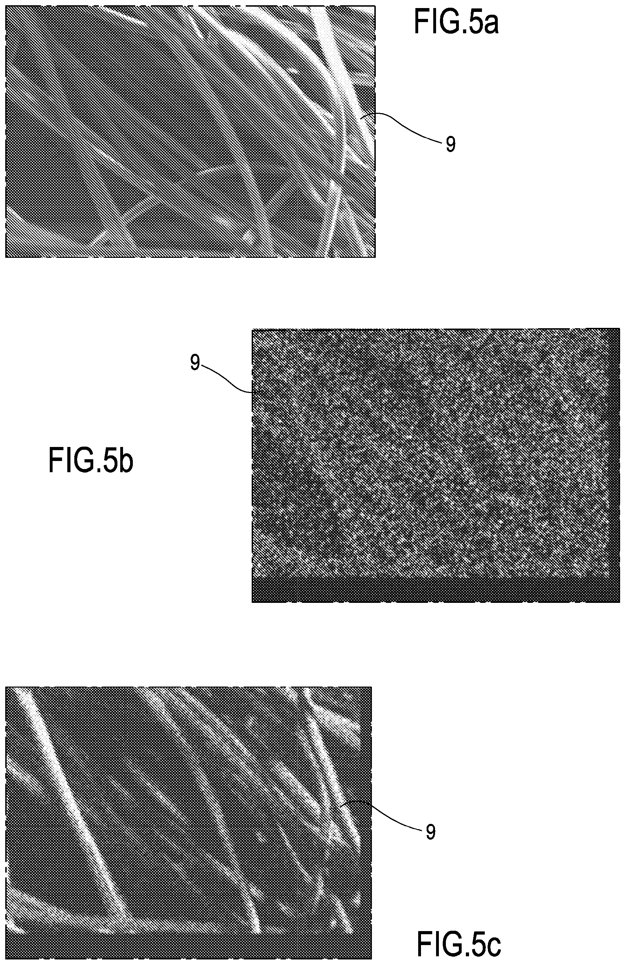

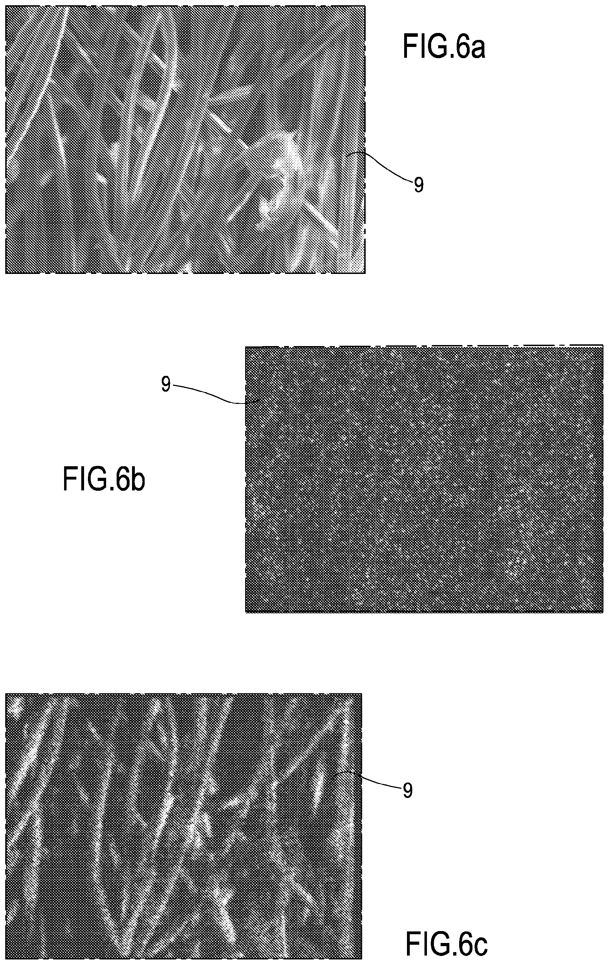

The Figures from 5a to 5c and from 6a to 6c provide immediate views, obtained by scanning electron microscope (SEM), of the effects of the method according to the present invention when applied on the article 9 made of nonwoven fabric (NWF) of the Figures from 4a to 4c, the adjustment parameters listed above (distance between flame 23 and surface 9p, concentration of the precursor compound in the solution, quantity of comburent in the reactor 8) having been defined to obtain the deposit on the surface 9p of the article 9 of a homogeneous layer of particles of titanium dioxide (TiO2) crystallised in the form of anatase and having a size of approximately 4nm. The Figures from 5a to 5c refer to an article 9 treated with the method according to the present invention with a periodic exposure of duration t_{des} equal to 150 seconds, while the Figures from 6a to 6c refer to an article 9 treated with the method according to the present invention with a periodic exposure of duration t_{des} of 180 seconds. Figures 5b and 6b show the distribution of titanium respectively on the details of Figures 5a and 6a, while Figures 5c and 6c show the distribution of oxygen respectively on the details of Figures 5a and 6a. As is readily apparent from the views of the Figures from 5a to 5c and from 6a to 6c, titanium dioxide is uniformly deposited on the fibres of the article 9 with a periodic exposure of duration t_{des} equal to 150 seconds, and still more with a periodic exposure of duration t_{des} equal to 180 seconds.

To enhance the antifungal and/or antibacterial properties imparted to the article 9 by the generation of metal oxide particles, in particular titanium dioxide (TiO2), by flame synthesis starting from a precursor compound and subsequent deposit by thermophoresis of the metal oxide particles thus generated on the surface 9p of the article 9, the surface 9p of the article 9 can subsequently be irradiated with ultraviolet radiation (UV), advantageously for a duration between 1 minute and 20 minute, preferably between 3 minutes and 10 minutes, still more preferably approximately 5 minutes. In this way, fungi and/or bacteria are eradicated not only by the sanitising and/or cleansing effect of the titanium dioxide (TiO2) particles, but also by the universally recognised sterilising properties of ultraviolet rays. Ultraviolet (UV) radiation produces a synergic effect to that of the metal oxide particles.

Laboratory tests were carried out to assess the effectiveness of the method according to the present invention, concretely verifying the antifungal and/or antibacterial properties provided to the article 9. In these tests, microorganisms were directly inoculated on the article 9, said article being in particular a sterile gauze of nonwoven fabric (NWF). The ability of the microorganisms to adhere to the fibres of the article 9 was then measured, both immediately after inoculation (to verify the resistance of the article 9, treated with the method according to the present invention, to the aggression of fungi and/or bacteria), and after 18 hours (to verify the persistence of the antifungal and/or antibacterial properties provided to the article 9 by the method according to the present invention).

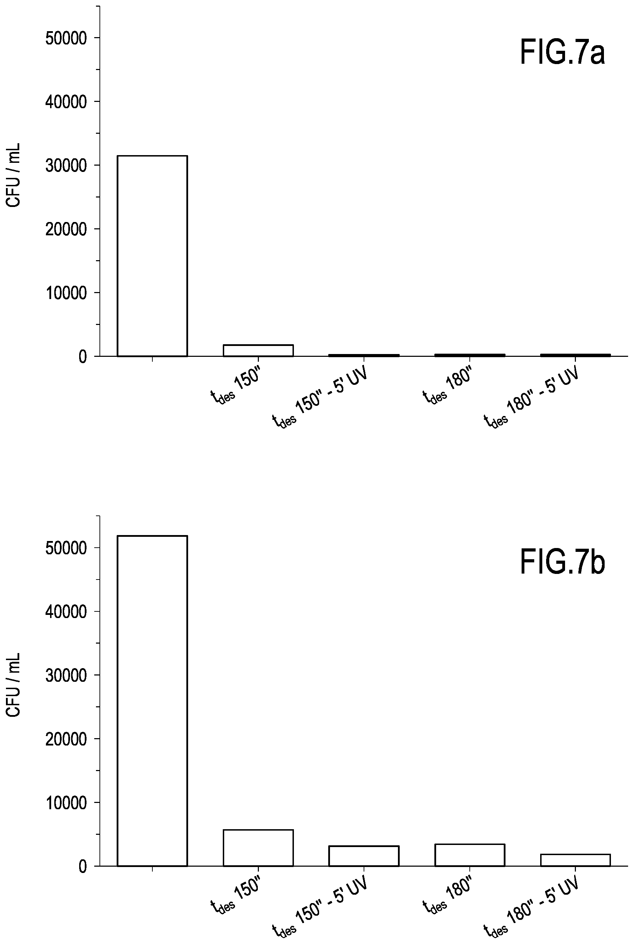

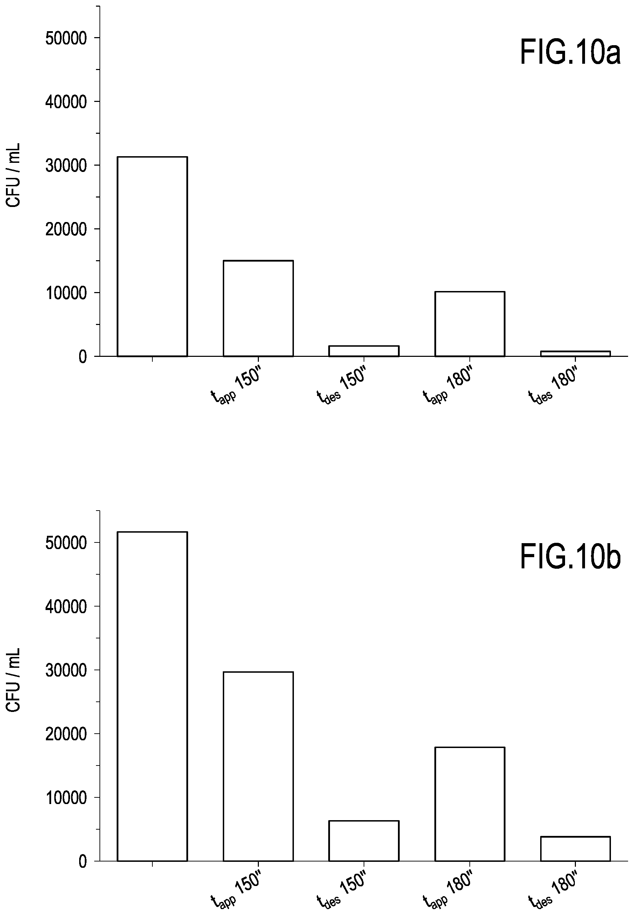

A first laboratory test was carried out to verify the antifungal properties provided to the article 9 by means of the method according to the present invention. The test was carried out using Candida albicans as test fungus. The results of this first test are immediately visible by means of the histograms of Figures 7a, 7b, 10a and 10b. From the histogram represented in Figure 7a it is noted that, when a charge of Candida albicans exceeding 30000 CFU/mL is inoculated (first column), less than one tenth of this charge is then actually able to adhere to the article 9. Excellent results are already obtained with a periodic exposure to the flame 23 of duration t_{des} equal to 150 seconds (second column). Still better results are obtained by following the treatment with irradiation with ultraviolet (UV) radiation for a duration of 5 minutes (third column), or prolonging the periodic exposure to a duration t_{des} of 180 seconds (fourth column), or prolonging the periodic exposure to the flame 23 to a duration t_{des} of 180 seconds and hence following the treatment with irradiation with ultraviolet (UV) radiation for a duration of 5 minutes (fifth column). From the histogram in Figure 7b it is appreciated that, while in the untreated sample the charge of Candida albicans reaches, after 18 hours, a value exceeding 50000 CFU/mL (first column), less than one tenth of said charge is found in the sample of article 9 treated with a periodic exposure to the flame 23 of duration t_{des} equal to 150 seconds (second column). Still better results are found in the sample subjected to the treatment according to the present invention was followed by an irradiation with ultraviolet (UV) radiation for a duration of 5 minutes (third column), or in the sample in which the periodic exposure to the flame 23 was prolonged to a duration t_{des} of 180 seconds (fourth column), or in the sample in which the periodic exposure to the flame 23 was prolonged to a duration t_{des} of 180 seconds and then the treatment according to the invention was followed by an irradiation with an ultraviolet radiation (UV) for a duration of 5 minutes (fifth column).

Figures 10a and 10b represent, in the form of histograms, the effectiveness of the method according to the present invention in relation to the Candida albicans fungus, comparing it with the effectiveness of the method according to the present invention (local application of particles of titanium dioxide by evaporation of the solvent) in relation to the same fungus. Comparison between the second column and the third column in Figure 10 shows that the local application of titanium dioxide particles for a duration tₐₚₚ of 150 seconds is able nearly to halve the charge (second column), while a periodic exposure with the method according to the present invention for a duration t_{des} equal to 150 seconds is able to reduce the charge by approximately ten times (third column). Comparison between the fourth column and the fifth column in Figure 10 shows that the local application of titanium dioxide particles for a duration tₐₚₚ of 180 seconds is able nearly to reduce to one third the charge (fourth column), while a periodic exposure with the method according to the present invention for a duration t_{des} equal to 180 seconds is able to reduce the charge by approximately ten times (fifth column).

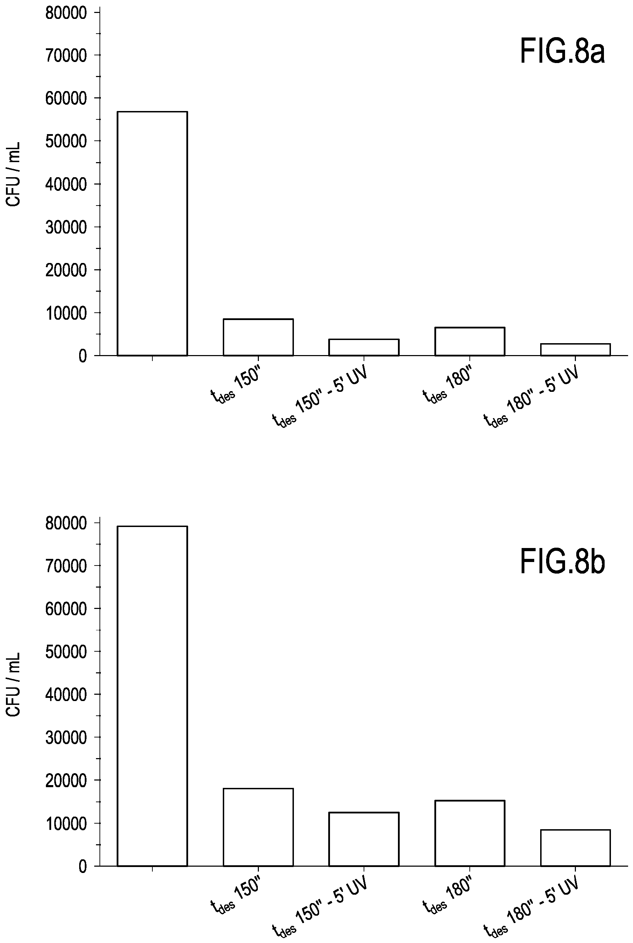

A second laboratory test was carried out to verify the antibacterial properties provided to the article 9 by means of the method according to the present invention, a Gram-positive bacterium (Staphylococcus aureus) being used for the second laboratory test. The results of this second test are immediately visible by means of the histograms of Figures 8a and 8b. From the histogram represented in Figure 8a it is noted that, when a charge of Staphylococcus aureus exceeding 55000 CFU/mL is inoculated (first column), less than one fifth of this charge is then actually able to adhere to the article 9. Excellent results are already obtained with a periodic exposure to the flame 23 of duration t_{des} equal to 150 seconds (second column). Still better results are obtained by following the treatment with irradiation with ultraviolet (UV) radiation for a duration of 5 minutes (third column), or prolonging the periodic exposure to a duration t_{des} of 180 seconds (fourth column), or prolonging the periodic exposure to the flame 23 to a duration t_{des} of 180 seconds and hence following the treatment with irradiation with ultraviolet (UV) radiation for a duration of 5 minutes (fifth column). From the histogram in Figure 8b it is appreciated that, while in the untreated sample the charge of Staphylococcus aureus reaches, after 18 hours, a value of approximately 80000 CFU/mL (first column), less than one quarter of said charge is found in the sample of article 9 treated with a periodic exposure to the flame 23 of duration t_{des} equal to 150 seconds (second column). Still better results are found in the sample subjected to the treatment according to the present invention was followed by an irradiation with ultraviolet (UV) radiation for a duration of 5 minutes (third column), or in the sample in which the periodic exposure to the flame 23 was prolonged to a duration t_{des} of 180 seconds (fourth column), or in the sample in which the periodic exposure to the flame 23 was prolonged to a duration t_{des} of 180 seconds and then the treatment according to the invention was followed by an irradiation with an ultraviolet radiation (UV) for a duration of 5 minutes (fifth column).

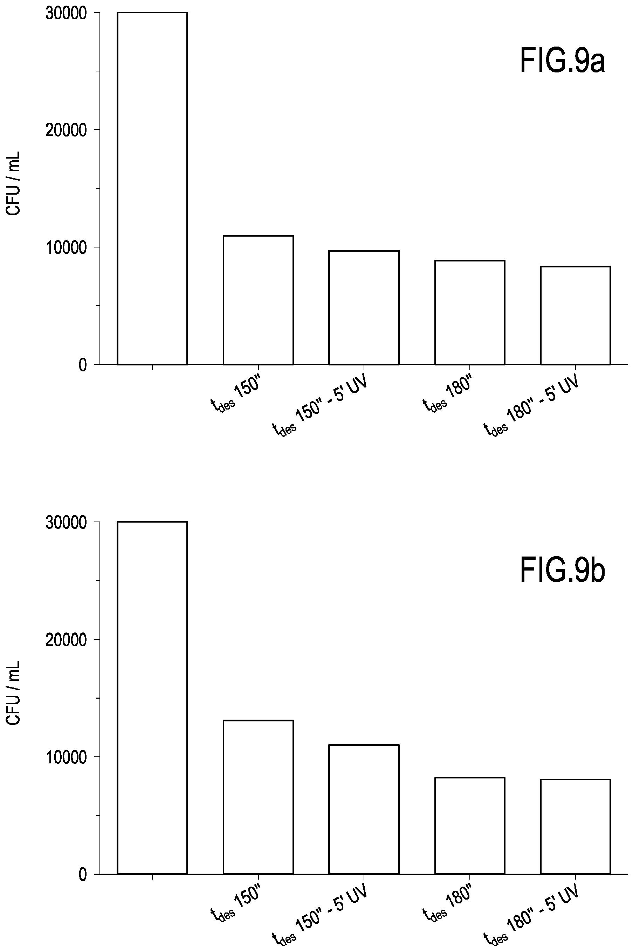

A third laboratory test was carried out to verify the antibacterial properties provided to the article 9 by means of the method according to the present invention, a Gram-negative bacterium (Pseudomonas aeruginosa) being used for the second laboratory test. The results of this third test are immediately visible by means of the histograms of Figures 9a and 9b. From the histogram represented in Figure 9a it is noted that, when a charge of Pseudomonas aeruginosa of approximately 30000 CFU/mL is inoculated (first column), approximately one third of this charge is then actually able to adhere to the article 9. Interesting results are already obtained with a periodic exposure to the flame 23 of duration t_{des} equal to 150 seconds (second column). Still better results are obtained by following the treatment with irradiation with ultraviolet (UV) radiation for a duration of 5 minutes (third column), or prolonging the periodic exposure to a duration t_{des} of 180 seconds (fourth column), or prolonging the periodic exposure to the flame 23 to a duration t_{des} of 180 seconds and hence following the treatment with irradiation with ultraviolet (UV) radiation for a duration of 5 minutes (fifth column). From the histogram in Figure 9b it is appreciated that, after 18 hours, less than half of said charge is found in the sample of article 9 treated with a periodic exposure to the flame 23 of duration t_{des} equal to 150 seconds (second column). Still better results are found in the sample subjected to the treatment according to the present invention was followed by an irradiation with ultraviolet (UV) radiation for a duration of 5 minutes (third column), or in the sample in which the periodic exposure to the flame 23 was prolonged to a duration t_{des} of 180 seconds (fourth column), or in the sample in which the periodic exposure to the flame 23 was prolonged to a duration t_{des} of 180 seconds and then the treatment according to the invention was followed by an irradiation with an ultraviolet radiation (UV) for a duration of 5 minutes (fifth column).

The antifungal and/or antibacterial activity of metal oxide particles, in particular titanium dioxide (TiO2), created by flame synthesis and then deposited on the surface 9p of the article 9 by means of the physical phenomenon of thermophoresis is thus far higher than the antifungal and/or antibacterial activity of the particles of metal oxide, in particular of titanium dioxide (TiO2), obtained by local evaporation of a solvent. This benefit of the method according to the present invention is due to the smaller size of the particles together with the lower presence of agglomerates.

In addition to the fundamental advantage of markedly increasing the antibacterial and/or antifungal properties of the particles of metal oxide, in particular of titanium dioxide (TiO2), the present invention achieves additional important advantages. The method according to the invention does not produce effluents and therefore has a limited impact on the environment. The method according to the invention is easily used for mass production of fabrics with antifungal and/or antibacterial properties. The method according to the invention is fast and assures high efficiency. The method according to the invention is suitable for multiple implementations on different fibrous materials (fabric, paper, ceramic, metal, synthetic and so on). The method according to the invention does not require pre-treatments and-or posttreatments of the fibrous material. The method according to the invention markedly improves the ability of the layer of titanium dioxide (TiO2) to withstand mechanical stresses (for example, friction and/or rubbing) and also appreciably improves the ability of the layer of titanium dioxide (TiO2) to withstand chemical agents (for example washing agents). Lastly, the method according to the invention does not alter the colour of the article to which the layer of titanium dioxide (TiO2) is applied.

The advantages listed above, together with additional significant advantages, are fully achieved by means of the method according to the present invention. These advantages are further achieved by an article, not covered by the claims, made of fibrous material treated with the method according to the present invention, the fibrous material comprising a synthetic material, preferably polypropylene and/or viscose, or cotton or paper and/or comprising a nonwoven fabric (NWF) and/or comprising a ceramic material or a metallic material. Lastly, these advantages are achieved by a medical and/or health care and/or personal care device, for example a sheet, a white coat, a mask, a bonnet, a bandage, a gauze, comprising an article, not covered by the claims, made of fibrous material treated with the method according to the present invention. According to another aspect of the present invention, to make treatment of the article 9 made of fibrous material easier and faster, the exposure of the surface 9p of the article 9 to the output section 8s of the reactor 8 can be obtained by a continuous exposure process. In this case, while the surface 9p of the article 9 is exposed to the output section 8s of the reactor 8, a surface of the article 9 not exposed to the output section 8s (typically the surface of the article 9 opposite to the surface 9p, the article 9 being a fundamentally two-dimensional object) is subjected to cooling. Said cooling allows the achievement of equally significant advantages. In the first place, said cooling preserves the article 9 from possible damages due to its exposure to the flame 23, avoiding structural modifications to the fibres that comprise the article 9. In the second place, said cooling acts as promoter of the thermophoretic transport of the metal oxide particles. Said cooling not only determines an increase of the temperature difference between the flame 23 and the article 9, but also promotes the penetration of the metal oxide particles in the article 9, the latter being made of fibrous material. According to a first embodiment, said cooling can be obtained using a Peltier cell. According to a second embodiment, said cooling can be obtained using a water or liquid cooling circuit.

## Claims

1. Method for treating an article (9) made of fibrous material, able to provide said article (9) with antibacterial and/or antifungal properties by the application on a surface (9p) of said article (9) of particles of a metal oxide, said method comprising the steps of:
i) generating said particles by flame synthesis of said particles from a precursor compound and
ii) depositing in a controlled manner said particles on a surface (9p) of said article (9) by thermophoresis.

2. Method according to claim 1, wherein said metal oxide is a titanium- or zinc- or iron- or silver-based oxide.

3. Method according to claim 2, wherein said metal oxide is titanium dioxide (TiO2),
wherein in particular said precursor compound is titanium tetraisopropoxide (TTIP) and/or titanium tetrachloride (TiCl4) and/or
wherein in particular said particles are deposited on said surface (9p) of said article (9) crystallised in the tetragonal crystalline system, said particles being deposited on said surface (9p) of said article (9) in the form of anatase by operating the flame (23) in conditions of excess comburent.

4. Method according to any of the claims from 1 to 3, wherein said particles are deposited on said surface (9p) of said article (9) in the condition of non-agglomeration.

5. Method according to any of the claims from 1 to 4, wherein said particles are deposited on said surface (9p) of said article (9) in a dimension between 1 nm and 50 nm,
wherein in particular said particles are deposited on said surface (9p) of said article (9) in a dimension between 3 nm and 10 nm,
wherein preferably said particles are deposited on said surface (9p) of said article (9) in a dimension between 4 nm and 5 nm.

6. Method according to any of the claims from 1 to 5, wherein said flame synthesis takes place by means of a reactor (9) to which said precursor compound is supplied and wherein said surface (9p) of said article (9) is exposed to the output section (8s) of said reactor (8).

7. Method according to claim 6, wherein the exposure of said surface (9p) of said article (9) to said output section (8s) of said reactor (8) is achieved by a process of periodic exposure in which a first phase of exposure is alternated to a second phase of non-exposure, the duration of said first phase of exposure being appreciably shorter than the duration of said second phase of non-exposure,
wherein in particular said duration of said first phase of exposure is shorter than 1 second,
wherein preferably said duration of said first phase of exposure is shorter than 0.1 seconds,
wherein more preferably said duration of said first phase of exposure is approximately 0.02 seconds and/or
wherein in particular said process of periodic exposure has a duration (t_{des}). between 10 seconds and 10 minutes,
wherein preferably said process of periodic exposure has a duration (t_{des}). between 1 minutes and 5 minutes,
wherein more preferably said process of periodic exposure has a duration (t_{des}) between 150 seconds and 180 seconds
and/or
wherein in particular said method of periodic exposure comprises rotating a carrousel (7), said article (9) being applied to said carrousel (7) so as to assure the exposure of said surface (9p) of said article (9) to said output section (8s) of said reactor (8).

8. Method according to claim 6, wherein the exposure of said surface (9p) of said article (9) to said output section (8s) of said reactor (8) is achieved by a process of continuous exposure,
wherein in particular, in said process of continuous exposure, a surface of said article (9) not exposed to said output section (8s) is subjected to cooling,
wherein preferably said cooling uses a Peltier cell or
wherein preferably said cooling uses a water cooling or liquid cooling circuit.

9. Method according to any of the claims from 6 to 8, wherein said precursor compound is supplied to said reactor (8) through a solution of said precursor compound in a solvent,
wherein in particular said solvent is ethanol or
wherein said precursor compound is supplied to said reactor (8) in aerosol form,
wherein in particular said aerosol is generated by means of a vibrating orifice aerosol generator (VOAG) and/or
wherein in particular said aerosol is mixed in said reactor (8) with a fuel,
wherein preferably said fuel is ethylene pre-mixed with air or
wherein said precursor compound is supplied to said reactor (8) by saturation of a dispersing medium of said precursor compound and/or of said solution of said precursor compound in said solvent,
wherein in particular said dispersing medium is air or
wherein the temperature of the flame (23) at said output section (8s) of said reactor (8) is between 1000 K and 2500 K,
wherein in particular the temperature of the flame (23) at said output section (8s) of said reactor (8) is between 1500 K and 2000 K,
wherein preferably the temperature of the flame (23) at said output section (8s) of said reactor (8) is between 1700 K and 1800 K or
wherein the temperature difference between said article (9) and the flame (23) at said output section (8s) of said reactor (8) is between 400 K and 2200 K,
wherein in particular the temperature difference between said article (9) and the flame (23) at said output section (8s) of said reactor (8) is between 400 K and 1700 K,
wherein preferably the temperature difference between said article (9) and the flame (23) at said output section (8s) of said reactor (8) is between 400 K and 1500 K.

10. Method according to any of the preceding claims, wherein said step i) is preceded by the preliminary step of adjusting the numerousness of said particles and/or the coalescence of said particles and/or the possible agglomeration of said particles and/or the crystalline group of said particles.

11. Method according to claim 10, wherein in said preliminary step an adjustment of the distance (D) of said surface (9p) of said article (9) from said output section (8s) of said reactor (8) is carried out, an increase of said distance (D) determining an increase of the size of said particles and/or the passage of said particles from the condition of non-agglomeration to the condition of agglomeration.

12. Method according to claim 10, wherein in said preliminary step an adjustment of the component of the distance (D) of said surface (9p) of said article (9) from said output section (8s) of said reactor (8) is carried out, an increase of the component of said distance (D) determining an increase of the size of said particles and/or the passage of said particles from the condition of non-agglomeration to the condition of agglomeration.

13. Method according to any of the claims from 10 to 12, wherein in said preliminary step an adjustment of the concentration of said precursor compound in said solution is carried out, an increase of said concentration determining an increase of the numerousness of said particles and/or of the size of said particles and/or the passage of said particles from the condition of non-agglomeration to the condition of agglomeration and/or
wherein in said preliminary step an adjustment of the stoichiometry of the flame (23) is carried out, a change of the stoichiometry of the flame (23) determining a change of the quantity of oxygen present in the environment of the reactor (8) and hence of the crystalline group of said particles,
wherein in particular said adjustment of the stoichiometry of the flame (23) is carried out by adjusting the quantity of comburent and/or of fuel supplied to said reactor (8).

14. Method according to any of the preceding claims, wherein conditions of pre-mixed flame or flame with diffusion in turbulent flow are established.

15. Method according to any of the preceding claims, further comprising the step of:
iii) irradiating said surface (9p) of said article (9) with an ultraviolet radiation (UV),
wherein in particular said step iii) has a duration between 1 minute and 20 minutes,
wherein preferably said step iii) has a duration between 3 minutes and 10 minutes,
wherein more preferably said step iii) has a duration of approximately 5 minutes.

## Patentansprüche

1. Verfahren zur Behandlung eines Gegenstandes (9), welcher aus einem faserigen Material hergestellt ist, dazu geeignet, den Gegenstand (9) mit antibakteriellen und/oder antifungiellen Eigenschaften zu versehen, indem auf einer Fläche (9p) des Gegenstands (9) Partikel aus einem Metalloxid aufgebracht werden, wobei das Verfahren die folgenden Schritte umfasst:
i) Erzeugen der Partikel durch Flammensynthese der Partikel aus einer Vorläuferverbindung und
ii) Abscheiden der Partikel in einer gesteuerten Weise auf einer Fläche (9p) des Gegenstands (9) durch Thermophorese.

2. Verfahren nach Anspruch 1, wobei das Metalloxid ein Oxid auf Titan- oder Zink- oder Eisen- oder Silber-Basis ist.

3. Verfahren nach Anspruch 2, wobei das Metalloxid Titandioxid (TiO2) ist, wobei insbesondere die Vorläuferverbindung Titantetraisopropoxid (TTIP) und/oder Titantetrachlorid (TiCl4) ist und/oder
wobei insbesondere die Partikel auf der Fläche (9p) des Gegenstands (9) in dem tetragonalen kristallinen System kristallisiert abgeschieden werden, wobei die Partikel (9) auf der Fläche (9p) des Gegenstands (9) in der Form von Anatas abgeschieden werden, indem die Flamme (23) in Zuständen überschüssigen Verbrennungsmittels betrieben wird.

4. Verfahren nach einem der Ansprüche von 1 bis 3, wobei die Partikel auf der Fläche (9p) des Gegenstands (9) in dem Zustand einer Nicht-Agglomeration abgeschieden werden.

5. Verfahren nach einem der Ansprüche von 1 bis 4, wobei die Partikel auf der Fläche (9p) des Gegenstands (9) in einer Abmessung von zwischen 1 nm und 50 nm abgeschieden werden,
wobei insbesondere die Partikel auf der Fläche (9p) des Gegenstands (9) in einer Abmessung von zwischen 3 nm und 10 nm abgeschieden werden,
wobei vorzugsweise die Partikel auf der Fläche (9p) des Gegenstands (9) in einer Abmessung von zwischen 4 nm und 5 nm abgeschieden werden.

6. Verfahren nach einem der Ansprüche von 1 bis 5, wobei die Flammensynthese mittels eines Reaktors (9) erfolgt, welchem die Vorläuferverbindung zugeführt wird, wobei die Fläche (9p) des Gegenstands (9) dem Ausgangsabschnitt (8s) des Reaktors (8) ausgesetzt ist.

7. Verfahren nach Anspruch 6, wobei das Aussetzen der Fläche (9p) des Gegenstands (9) gegenüber dem Ausgangsabschnitt (8s) des Reaktors (8) durch einen Prozess eines periodischen Aussetzens erreicht wird, wobei eine erste Phase eines Aussetzens mit einer zweiten Phase eines Nicht-Aussetzens abgewechselt wird, wobei die Dauer der ersten Phase eines Aussetzens wesentlich kürzer als die Dauer der zweiten Phase eines Nicht-Aussetzens ist,
wobei insbesondere die Dauer der ersten Phase eines Aussetzens kürzer als 1 Sekunde ist,
wobei vorzugsweise die Dauer der ersten Phase eines Aussetzens kürzer als 0,1 Sekunde ist,
wobei weiter vorzugsweise die Dauer der ersten Phase eines Aussetzens etwa 0,02 Sekunden beträgt, und/oder
wobei insbesondere der Prozess eines periodischen Aussetzens eine Dauer (t_{des}) zwischen 10 Sekunden und 10 Minuten aufweist,
wobei vorzugsweise der Prozess eines periodischen Aussetzens eine Dauer (t_{des}) zwischen 1 Minute und 5 Minuten aufweist,
wobei weiter vorzugsweise der Prozess eines periodischen Aussetzens eine Dauer (t_{des}) zwischen 150 Sekunden und 180 Sekunden aufweist,
und/oder
wobei insbesondere das Verfahren eines periodischen Aussetzens ein Rotieren eines Karussells (7) umfasst, wobei der Gegenstand (9) so an dem Karussell (7) angebracht wird, dass das Aussetzen der Fläche (9p) des Gegenstands (9) gegenüber dem Ausgangsabschnitt (8s) des Reaktors (8) sichergestellt wird.

8. Verfahren nach Anspruch 6, wobei das Aussetzen der Fläche (9p) des Gegenstands (9) gegenüber dem Ausgangsabschnitt (8s) des Reaktors (8) durch einen Prozess eines kontinuierlichen Aussetzens erreicht wird,
wobei insbesondere, bei dem Prozess eines kontinuierlichen Aussetzens, eine Fläche des Gegenstands (9), welche dem Ausgangsabschnitt (8s) nicht ausgesetzt ist, einem Kühlen unterzogen wird,
wobei vorzugsweise das Kühlen ein Peltier-Element verwendet oder
wobei vorzugsweise das Kühlen eine Wasserkühlung oder einen Flüssig-Kühlkreislauf verwendet.

9. Verfahren nach einem der Ansprüche von 6 bis 8, wobei die Vorläuferverbindung dem Reaktor (8) durch eine Lösung der Vorläuferverbindung in einem Lösungsmittel zugeführt wird,
wobei insbesondere das Lösungsmittel Ethanol ist oder
wobei die Vorläuferverbindung dem Reaktor (8) in Aerosol-Form zugeführt wird, wobei insbesondere das Aerosol mittels eines Aerosolgenerators mit vibrierender Öffnung (VOAG) erzeugt wird, und/oder
wobei insbesondere das Aerosol in dem Reaktor (8) mit einem Brennstoff gemischt wird, wobei vorzugsweise der Brennstoff Ethylen vorgemischt mit Luft ist, oder
wobei die Vorläuferverbindung den Reaktor (8) durch Saturation eines dispergierenden Mediums der Vorläuferverbindung und/oder der Lösung der Vorläuferverbindung in dem Lösungsmittel zugeführt wird,
wobei insbesondere das dispergierende Medium Luft ist, oder
wobei die Temperatur der Flamme (23) an dem Ausgangsabschnitt (8s) des Reaktors (8) zwischen 1000 K und 2500 K beträgt,
wobei insbesondere die Temperatur der Flamme (23) an dem Ausgangsabschnitt (8s) des Reaktors (8) zwischen 1500 K und 2000 K beträgt,
wobei vorzugsweise die Temperatur der Flamme (23) an dem Ausgangsabschnitt (8s) des Reaktors (8) zwischen 1700 K und 1800 K beträgt, oder
wobei die Temperaturdifferenz zwischen dem Gegenstand (9) und der Flamme (23) an dem Ausgangsabschnitt (8s) des Reaktors (8) zwischen 400 K und 2200 K beträgt, wobei insbesondere die Temperaturdifferenz zwischen dem Gegenstand (9) und der Flamme (23) an dem Ausgangsabschnitt (8s) des Reaktors (8) zwischen 400 K und 1700 K beträgt,
wobei vorzugsweise die Temperaturdifferenz zwischen dem Gegenstand (9) und der Flamme (23) an dem Ausgangsabschnitt (8s) des Reaktors (8) zwischen 400 K und 1500 K beträgt.

10. Verfahren nach einem der vorhergehenden Ansprüche, wobei dem Schritt i) der vorrausgehende Schritt eines Einstellens der Anzahl der Partikel und/oder der Koaleszenz der Partikel und/oder der möglichen Agglomeration der Partikel und/oder der kristallinen Gruppe der Partikel vorausgeht.

11. Verfahren nach Anspruch 10, wobei in dem vorausgehenden Schritt eine Einstellung des Abstands (D) der Fläche (9p) des Gegenstands (9) von dem Ausgangsabschnitt (8s) des Reaktors (8) ausgeführt wird, wobei eine Zunahme des Abstandes (D) eine Zunahme der Größe der Partikel und/oder den Übergang der Partikel von dem Zustand einer Nicht-Agglomeration zu dem Zustand einer Agglomeration bestimmt.

12. Verfahren nach Anspruch 10, wobei in dem vorausgehenden Schritt eine Einstellung der Komponente des Abstands (D) der Fläche (9p) des Gegenstands (9) von dem Ausgangsabschnitt (8s) des Reaktors (8) ausgeführt wird, wobei eine Zunahme der Komponente des Abstandes (D) eine Zunahme der Größe der Partikel und/oder den Übergang der Partikel von dem Zustand einer Nicht-Agglomeration zu dem Zustand einer Agglomeration bestimmt.

13. Verfahren nach einem der Ansprüche von 10 bis 12, wobei in dem vorausgehenden Schritt eine Einstellung der Konzentration der Vorläuferverbindung in der Lösung ausgeführt wird, wobei eine Zunahme der Konzentration eine Zunahme der Anzahl der Partikel und/oder der Größe der Partikel und/oder den Übergang der Partikel von dem Zustand einer Nicht-Agglomeration zu dem Zustand einer Agglomeration bestimmt und/oder
wobei in dem vorausgehenden Schritt eine Einstellung der Stöchiometrie der Flamme (23) ausgeführt wird, wobei eine Änderung der Stöchiometrie der Flamme (23) eine Änderung der Menge von Sauerstoff, welche in der Umgebung des Reaktors (8) vorhanden ist, und damit der kristallinen Gruppe der Partikel bestimmt,
wobei insbesondere die Einstellung der Stöchiometrie der Flamme (23) durch Einstellen der Menge eines Verbrennungsmittels und/oder eines Brennstoffs ausgeführt wird, welche dem Reaktor (8) zugeführt werden.

14. Verfahren nach einem der vorhergehenden Ansprüche, wobei Zustände einer vorgemischten Flamme oder einer Flamme mit einer Diffusion in turbulenter Strömung geschaffen werden.

15. Verfahren nach einem der vorhergehenden Ansprüche, ferner umfassend den folgenden Schritt:
iii) Bestrahlen der Fläche (9p) des Gegenstands (9) mit einer Ultraviolett-Strahlung (UV),
wobei insbesondere der Schritt iii) eine Dauer von zwischen 1 Minute und 20 Minuten aufweist,
wobei vorzugsweise der Schritt iii) eine Dauer von zwischen 3 Minuten und 10 Minuten aufweist,
wobei weiter vorzugsweise der Schritt iii) eine Dauer von etwa 5 Minuten aufweist.

## Revendications

1. Procédé de traitement d'un article (9) constitué d'un matériau fibreux, capable de fournir audit article (9) des propriétés antibactériennes et/ou antifongiques par l'application sur une surface (9p) dudit article (9) des particules d'un oxyde métallique, ledit procédé comprenant les étapes de :
i) génération desdites particules par synthèse à la flamme desdites particules à partir d'un composé précurseur et
ii) dépôt d'une manière contrôlée desdites particules sur une surface (9p) dudit article (9) par thermophorèse.

2. Procédé selon la revendication 1, ledit oxyde métallique étant un oxyde à base de titane- ou de zinc- ou de fer- ou d'argent.

3. Procédé selon la revendication 2, ledit oxyde métallique étant le dioxyde de titane (TiO₂), en particulier ledit composé précurseur étant le tétraisopropoxyde de titane (TTIP) et/ou le tétrachlorure de titane (TiCl₄) et/ou
en particulier lesdites particules étant déposées sur ladite surface (9p) dudit article (9) cristallisé dans le système cristallin tétragonal, lesdites particules étant déposées sur ladite surface (9p) dudit article (9) sous la forme anatase en faisant fonctionner la flamme (23) sous des conditions de comburant en excès.

4. Procédé selon l'une quelconque des revendications de 1 à 3, lesdites particules étant déposées sur ladite surface (9p) dudit article (9) dans la condition sans agglomération.

5. Procédé selon l'une quelconque des revendications de 1 à 4, lesdites particules étant déposées sur ladite surface (9p) dudit article (9) dans une dimension comprise entre 1 nm et 50 nm,
en particulier lesdites particules étant déposées sur ladite surface (9p) dudit article (9) dans une dimension comprise entre 3 nm et 10 nm,
préférablement lesdites particules étant déposées sur ladite surface (9p) dudit article (9) dans une dimension comprise entre 4 nm et 5 nm.

6. Procédé selon l'une quelconque des revendications de 1 à 5, ladite synthèse à la flamme ayant lieu à l'aide d'un réacteur (9) auquel ledit composé précurseur est alimenté et ladite surface (9p) dudit article (9) étant exposée à la section de sortie (8s) dudit réacteur (8).

7. Procédé selon la revendication 6, l'exposition de ladite surface (9p) dudit article (9) à ladite section de sortie (8s) dudit réacteur (8) étant atteinte par un procédé d'exposition périodique dans lequel une première phase d'exposition est alternée vers une seconde phase de non-exposition, la durée de ladite première phase d'exposition étant de manière appréciable plus courte que la durée de ladite seconde phase de non-exposition,
en particulier ladite durée de ladite première phase d'exposition étant plus courte que 1 seconde,
préférablement ladite durée de ladite première phase d'exposition étant plus courte que 0,1 seconde,
plus préférablement ladite durée de ladite première phase d'exposition étant d'approximativement 0,02 seconde et/ou
en particulier ledit procédé d'exposition périodique ayant une durée (t_{des}) comprise entre 10 secondes et 10 minutes,
préférablement ledit procédé d'exposition périodique ayant une durée (t_{des}) comprise entre 1 minute et 5 minutes,
plus préférablement ledit procédé d'exposition périodique ayant une durée (t_{des}) comprise entre 150 secondes et 180 secondes et/ou
en particulier ledit procédé d'exposition périodique comprenant la rotation d'un carrousel (7), ledit article (9) étant appliqué audit carrousel (7) afin d'assurer l'exposition de ladite surface (9p) dudit article (9) à ladite section de sortie (8s) dudit réacteur (8).

8. Procédé selon la revendication 6, l'exposition de ladite surface (9p) dudit article (9) à ladite section de sortie (8s) dudit réacteur (8) étant atteinte par un procédé d'exposition continue,
en particulier, dans ledit procédé d'exposition continue, une surface dudit article (9) non exposée à ladite section de sortie (8s) étant soumise à un refroidissement, préférablement ledit refroidissement utilisant une cellule de Peltier ou
préférablement ledit refroidissement utilisant un circuit de refroidissement à l'eau ou de refroidissement liquide.

9. Procédé selon l'une quelconque des revendications de 6 à 8, ledit composé précurseur étant fourni audit réacteur (8) à travers une solution dudit composé précurseur dans un solvant,
en particulier ledit solvant étant l'éthanol ou
ledit composé précurseur étant alimenté audit réacteur (8) sous une forme d'aérosol,
en particulier ledit aérosol étant généré à l'aide d'un générateur d'aérosol à orifice vibrant (VOAG) et/ou
en particulier ledit aérosol étant mélangé dans ledit réacteur (8) avec un combustible,
préférablement ledit combustible étant l'éthylène pré-mélangé avec de l'air ou
ledit composé précurseur étant alimenté audit réacteur (8) par saturation d'un milieu de dispersion dudit composé précurseur et/ou de ladite solution dudit composé précurseur dans ledit solvant,
en particulier ledit milieu de dispersion étant l'air ou
la température de la flamme (23) au niveau de ladite section de sortie (8s) dudit réacteur (8) étant comprise entre 1 000 K et 2 500 K,
en particulier la température de la flamme (23) au niveau de ladite section de sortie (8s) dudit réacteur (8) étant comprise entre 1 500 K et 2 000 K,
préférablement la température de la flamme (23) au niveau de ladite section de sortie (8s) dudit réacteur (8) étant comprise entre 1 700 K et 1 800 K ou
la différence de température entre ledit article (9) et la flamme (23) au niveau de ladite section de sortie (8s) dudit réacteur (8) étant comprise entre 400 K et 2 200 K,
en particulier la différence de température entre ledit article (9) et la flamme (23) au niveau de ladite section de sortie (8s) dudit réacteur (8) étant comprise entre 400 K et 1 700 K,
préférablement la différence de température entre ledit article (9) et la flamme (23) au niveau de ladite section de sortie (8s) dudit réacteur (8) étant comprise entre 400 K et 1 500 K.

10. Procédé selon l'une quelconque des revendications précédentes, ladite étape i) étant précédée par l'étape préliminaire d'ajustement du grand nombre desdites particules et/ou de la coalescence desdites particules et/ou de l'agglomération possible desdites particules et/ou du groupe cristallin desdites particules.

11. Procédé selon la revendication 10, dans ladite étape préliminaire, un ajustement de la distance (D) de ladite surface (9p) dudit article (9) à partir de ladite section de sortie (8s) dudit réacteur (8) étant effectué, une augmentation de ladite distance (D) déterminant une augmentation de la taille desdites particules et/ou le passage desdites particules depuis l'état de non-agglomération vers l'état d'agglomération.

12. Procédé selon la revendication 10, dans ladite étape préliminaire, un ajustement de la composante de la distance (D) de ladite surface (9p) dudit article (9) à partir de ladite section de sortie (8s) dudit réacteur (8) étant effectué, une augmentation de la composante de ladite distance (D) déterminant une augmentation de la taille desdites particules et/ou le passage desdites particules depuis l'état de non-agglomération vers l'état d'agglomération.

13. Procédé selon l'une quelconque des revendications de 10 à 12, dans ladite étape préliminaire, un ajustement de la concentration dudit composé précurseur dans ladite solution étant effectué, une augmentation de ladite concentration déterminant une augmentation du grand nombre desdites particules et/ou de la taille desdites particules et/ou du passage desdites particules depuis l'état de non-agglomération vers l'état d'agglomération et/ou
dans ladite étape préliminaire, un ajustement de la stœchiométrie de la flamme (23) étant effectué, un changement de la stœchiométrie de la flamme (23) déterminant un changement de la quantité d'oxygène présent dans l'environnement du réacteur (8) et de là du groupe cristallin desdites particules,
en particulier ledit ajustement de la stœchiométrie de la flamme (23) étant effectué par ajustement de la quantité de comburant et/ou de combustible alimenté au niveau dudit réacteur (8).

14. Procédé selon l'une quelconque des revendications précédentes, les conditions de flamme pré-mélangée ou de flamme avec diffusion dans un écoulement turbulent étant établies.

15. Procédé selon l'une quelconque des revendications précédentes, comprenant en outre l'étape de :
iii) irradiation de ladite surface (9p) dudit article (9) avec un rayonnement ultraviolet (UV),
en particulier ladite étape iii) ayant une durée comprise entre 1 minute et 20 minutes,
préférablement ladite étape iii) ayant une durée comprise entre 3 minutes et 10 minutes,
plus préférablement ladite étape iii) ayant une durée d'approximativement 5 minutes.
